# EUROPÄISCHE PATENTSCHRIFT

(11) **EP 4 326 177 B1**
(45) Veröffentlichungstag und Bekanntmachung des Hinweises auf die Patenterteilung: **18.03.2026**
(21) Anmeldenummer: 22719873.6
(22) Anmeldetag: 30.03.2022
(51) Int. Cl.: A61B 17/70, A61B 17/86

(54) **TEMPORÄR FIXIERBARE OSTEOSYNTHESEVORRICHTUNG FÜR WIRBEL MIT DREHBAREM FIXIER-ELEMENT**
TEMPORARILY FIXABLE OSTEOSYNTHESIS DEVICE FOR VERTEBRAE, WITH A ROTATABLE FIXING ELEMENT
DISPOSITIF D'OSTÉOSYNTHÈSE À FIXATION TEMPORAIRE POUR VERTÈBRES, AVEC UN ÉLÉMENT DE FIXATION ROTATIF

(30) Priorität: 22.04.2021 DE 102021002120
(43) Veröffentlichungstag der Anmeldung: 28.02.2024
(73) Patentinhaber: Ortho Hub Ventures UG (Haftungsbeschränkt), 70794 Filderstadt (DE)
(72) Erfinder: HEUER, Frank, 70794 Filderstadt (DE)
(74) Vertreter: Stolmár & Partner Patentanwälte PartG mbB
(86) Internationale Anmeldenummer: PCT/EP2022/058487
(87) Internationale Veröffentlichungsnummer: WO 2022/223255

(56) Entgegenhaltungen:
- EP-A1- 3 128 934
- WO-A1-2018/024414
- DE-A1- 102016 108 504
- DE-B3- 102018 102 173

## Beschreibung

### Stand der Technik

Dem Stand der Technik sind verschiedene Osteosynthesevorrichtungen zur Versorgung der Wirbelsäule, wie beispielsweise Pedikelschrauben, bekannt. Solche Osteosynthesevorrichtungen werden dafür verwendet Wirbelsäulenfehlstellungen zu korrigieren oder Frakturen zu stabilisieren, indem die Osteosynthesevorrichtungen in den Wirbelknochen eingebracht und befestigt werden und dann über Längsstäbe, oder sogenannten Verbindungsstäbe, miteinander verbunden werden, um die Wirbel in einer gewünschten Stellung miteinander zu fixieren. Dabei werden die Längsstäbe mit Hilfe von VerriegelungsElementen, wie beispielsweise Madenschrauben oder sonstigen Verschluss-Elementen, an den Osteosynthesevorrichtungen montiert und rutschfest fixiert. Als Osteosynthesevorrichtungen kommen vorzugsweise Pedikelschrauben zum Einsatz, die einen Knochenanker besitzen, welcher in wenigstens einer Ebene mit einem Gabelkopf verschwenkbar gelagert ist und bei fixierter Madenschraube winkelstabil ist. Als Knochenanker kommen vorzugsweise Knochenschrauben mit einem Kugelkopf zum Einsatz. Regulär werden Osteosynthesevorrichtungen mit Knochenanker und Gabelkopf so montiert, dass der Knochenanker von proximal kommend in den Gabelkopf durch die distale Öffnung des Gabelkopfes geführt wird. Das funktioniert nur, wenn der äußere Durchmesser des Knochenankerschafts kleiner ist als der Kugelkopfdurchmesser des Knochenankers und der äußere Durchmesser des Knochenankerschafts kleiner ist als der Durchmesser der distalen Öffnung des Gabelkopfes. Problematisch ist die Montage, wenn der äußere Durchmesser des Knochenankerschafts größer ist als der Öffnungsdurchmessers der Gabelkopfes und/oder des Kugelkopfdurchmesser des Knochenankers.

Aus dem Stand der Technik ist eine Pedikelschraube (DE102011053295A1) bekannt, welche temporär verriegelbar ist und somit ein erweitertes Anwendungssprektrum bei der Versorgung von Wirbelsäuleninstabilitäten erlaubt. Damit ist es möglich polyaxial bewegliche Pedikelschrauben in einer beliebigen Orientierung in den Wirbelknochen einzubringen und im Anschluss kann der Anwender den Winkel zwischen Gabelkopf und Knochenanker temporär verklemmen. Mit einer temporär gesperrten Polyaxialität ist es möglich, dass über den Gabelkopf Korrekturkräfte auf den Knochenanker eingeleitet werden und diese sich direkt auf die Stellung und Position der Wirbelknochen auswirkt. Ohne diese temporäre Verklemmung sind solche Korrekturmanöver nicht oder nur schwer ausführbar. Bei einer solchen Anordnung ist es erforderlich, dass das Druckstück mit Hilfe eines Instruments permanent geklemmt wird und das Instrument jederzeit an der Schraube befestigt ist, damit die für die temporäre Klemmung notwendige Kompressionskraft aufrechterhalten wird. Bei manchen Wirbelsäulenkorrekturmanövern, wie beispielsweise bei der Korrektur von Deformitäten und Skoliosen, ist kein Platz für derartige Instrumente. Deshalb wäre es wünschenswert, ein Schraubenimplantat vorzusehen, welches eigenständig in der Lage ist, die temporäre Klemmung aufrechtzuerhalten, sobald die Instrumente entfernt sind.

Aus der Anmeldung DE102018102173B3 ist ein Pedikelschraubenanker bekannt, bei dem die temporäre Kompressionskraft permanent aufrechterhalten bleibt, indem ein lösbares Stift-Element vorgesehen ist. Allerdings erfordert dieser Aufbau für die Erzeugung der temporären Klemmung eine hebelartige Betätigung des Druckstücks. Daraus resultiert eine erhöhte kombinierte Kompressions- und Biegebeanspruchung des Druckstücks was zu einer mechanischen Limitation der maximalen temporären Klemmwirkung führt. Des Weiteren ist es notwendig, dass das Druckstück in seiner Materialdicke so dimensioniert ist, dass diese Belastungen sich nicht zerstörend auswirken. Dies hat zur Folge, dass eine solche Pedikelschraube in ihrer Bauhöhe größer ist als eine reguläre Pedikelschraube. Deshalb ist es wünschenswert, dass die temporäre Klemmwirkung nicht über das Druckstück selbst eingeleitet wird, sondern direkt am Knochenanker-Kopfbereich angreift. Damit ist das Druckstück bei der temporären Klemmung mechanisch entkoppelt und die Osteosynthesevorrichtung kann Reserven bzgl. in der maximalen Klemmwirkung bereitstellen.

Des Weiteren ist zu erkennen, dass keine dieser temporär klemmbaren Pedikelschrauben-Konzepte in der Lage ist, Schraubenschäfte von distal kommend aufzunehmen. Mit ihnen können nur Knochenanker angewendet werden, bei denen der äußere Durchmesser des Knochenankerschafts kleiner ist als der Durchmesser der distalen Öffnung des Gabelkopfes.

Aufgabe der Erfindung ist es deshalb eine temporär klemmbare Osteosynthesevorrichtung, insbesondere Pedikelschraube, bereitzustellen, die es erlaubt, dass ein Knochenanker von distal kommend montierbar ist und dass das Druckstück bei Betätigung der temporären Klemmung nicht belastet, sondern erst bei finaler Verriegelung der Osteosynthesevorrichtung mit den Verbindungsstab und dem Verschluss-Element mit einer Druckkraft beaufschlagt wird. Dadurch ergibt sich einerseits eine Modularität des Schraubensystems, was Vorteile bzgl. der reduzierten Kapitalbindung beim Anwender hat und es kann ein insgesamt größeres Schrauben-Portfolio angeboten werden. Anderseits ergibt sich durch die Kraft-Fluss-Aufteilung bei der temporären Klemmung ohne die mechanische Beteiligung des Druckstücks ein deutlicher Stabilitätsgewinn gegenüber bisherigen Konzepten und die Pedikelschraube kann in ihrer Bauhöhe kleiner gestaltet werden. Gleichzeitig soll das erfindungsgemäße Konzept die temporär erzeugte Klemmung selbstständig aufrechterhalten, auch wenn sämtliche Instrumente entfernt sind.

### Darstellung der Erfindung

Diese Aufgabe wird gelöst durch eine Osteosynthesevorrichtung nach Anspruch 1. Weitere, die Erfindung ausgestaltende Merkmale sind in den Unteransprüchen enthalten.

Eine erfindungsgemäße Osteosynthesevorrichtung zur Behandlung der Wirbelsäule, weist einen in einer Seitenansicht u-förmigen Gabelkopf auf, welcher eine Durchgangsöffnung besitzt und wobei der Gabelkopf in proximaler Richtung zwei Gabelschenkel mit einem innenliegenden Gewinde aufweist, und darin ein Verbindungsstab aufgenommen werden kann, und im Gabelkopf in distaler Richtung in der Durchgangsöffnung ein Kugelkopfaufnahmebereich vorgesehen ist und darin ein Knochenanker schwenkbar gelagert ist, wobei am Gabelkopf eine Quer-Öffnung vorgesehen ist, welche mit der Durchgangsöffnung des Gabelkopfs über einen Wandungsausschnitt kommuniziert und in dieser Quer-Öffnung ein Fixier-Element um eine Quer-Öffnungs-Achse drehbar gelagert ist und das Fixier-Element mit Einleitung eines Torsionsmoments um die Quer-Öffnungs-Achse den Kopfbereich des Knochenankers winkelstabil im Gabelkopf klemmt, wobei das Zentrum des innenliegenden Gewindes und das Zentrum des Kugelkopfaufnahmebereichs eine Zentralachse definieren, und sich diese Zentralachse nicht mit der Quer-Öffnungs-Achse schneidet.

Die Erfindung betrifft eine Osteosynthesevorrichtung, insbesondere polyaxiale Pedikelschraube bestehend aus einem Kopf aufweisenden Knochenanker, einem in einer Seitenansicht u-förmigen Gabelkopf, einem darin befindlichen Druckstück, einem innenliegenden und in einer Quer-Öffnung geführtem Fixier-Element, wobei das Fixier-Element dazu geeignet ist, durch Rotation um die Quer-Öffnungs-Achse den Knochenanker-Kopfbereich temporär in allen Freiheitsgraden zu klemmen, ohne dass dabei ein Druckstück notwendig ist, oder falls vorhanden, nicht belastet wird.

In der bevorzugten Ausgestaltungsform ist es möglich den Klemmeffekt mittelbar über ein Stell-Element beziehungsweise unmittelbar über das selbst Fixier-Element einzuleiten ohne dass der Verbindungsstab oder die Madenschraube vorhanden sind. Da es sich nicht um eine finale Klemmung mit eingelegtem Verbindungsstab handelt, nennt sich diese Art der Klemmung, temporäre Klemmung. Mit der temporären Klemmung ist es für den Anwender möglich, während der Operation eine polyaxiale Schraube in einer gewünschten Winkelstellung in eine monoaxiale Schraube zu konvertieren. Das heißt, es sind alle rotatorischen Freiheitsgrade einer polyaxialen Schraube temporär gesperrt. Die Schraube verhält sich monoaxial. Damit kann der Anwender nun den zu behandelten Wirbel translatorisch als auch rotatorisch so lange manipulieren, bis er in der gewünschten Endstellung einen Verbindungsstabe einlegt und mit der Madenschraube fixiert. Mit einer polyaxialen Schraube sind solche Korrektionsmanöver nicht möglich, da eine von Patienten-seitig außen initiierte Korrektionsmanöver in einer freien Bewegung des polyaxial- Kugel-Gelenks resultiert und somit nicht an den Wirbel weitergeleitet wird. Dies funktioniert nur mit deaktivierten rotatorischen Freiheitsgraden im Kugelgelenk, also temporär geklemmt ist.

Unabhängig von der temporären Klemmung, muss nach der Implantation der Osteosynthesevorrichtung in den Knochen, ein Verbindungsstab eingelegt und mit Hilfe eines Verschlusselements die Osteosynthesevorrichtung final in allen Freiheitsgraden fixiert werden. Dies geschieht durch Festschrauben des Verschlusselements.

Bei fest angezogenen Verschlusselement wird eine axiale Kompressionskraft vom Verschlusselement auf den Verbindungsstab übertragen, und dieser drückt auf die Stabauflagerstellen des Druckstücks und erzeugt eine minimale Relativbewegung des Druckstücks weiter nach distal, so dass der Knochenanker winkelstabil im Kugelsitz geklemmt wird. Optimalerweise werden zwei oder mehr Osteosynthesevorrichtungen mit Hilfe eines Verbindungsstabs miteinander verbunden.

Dabei ist der Gabelkopf so ausgestaltet, dass ein Verbindungsstab einlegbar und mit einem Verriegelungs-Element am Gabelkopf fixierbar ist. Dadurch wird wie bereits erwähnt eine winkelstabile Klemmung zwischen dem Knochenanker-Kopfbereich und Gabelkopf erzielt. Die winkelstabile Klemmung mit Hilfe des Verriegelung-Elements und die winkelstabile Klemmung mit Hilfe des Fixier-Elements funktionieren in dem erfindungsgemäßen Aufbau unabhängig voneinander. Sie sind getrennt aber auch in Kombination aktivierbar.

In einer bevorzugten Ausführungsform der Osteosynthesevorrichtung besitzt der Gabelkopf eine Durchgangsöffnung und formt in proximaler Richtung zwei Gabelschenkel mit einem innenliegenden Gewinde für ein Verriegelungs-Element aus. In distaler Richtung ist in der Durchgangsöffnung ein Kugelkopfaufnahmebereich vorgesehen, worin ein Knochenanker schwenkbar gelagert ist.

Als Knochenanker kommen vorzugsweise Knochenschrauben zum Einsatz, die mit einem Knochen verschraubbar sind. Es sind aber auch Haken, klingenähnliche Anker, Klemmen, Nägel und andersartig gestaltete Knochenanker anwendbar. Die wesentlichen Merkmale des Knochenankers sind ein kugelähnlicher Kopf, ein Halsbereich und ein Bereich, welcher im oder am Knochen verankert oder befestigt werden kann. In dieser Patentanmeldung soll hauptsächlich auf den Gabelkopf eingegangen werden, und mit Knochenanker sollen alle erdenklichen mit einem Knochen verbindbaren Elemente verstanden werden.

Ein wesentliches Merkmal der Erfindung ist, dass das Zentrum des innenliegenden Gewindes und das Zentrum des Kugelkopfaufnahmebereichs die Position und Orientierung der Zentralachse definiert. Seitlich und in einem Abstand, der größer ist als der Durchmesser des Verriegelung-Elements, ist eine Axial-Öffnung für ein Stell-Element vorgesehen. Die Axial-Öffnung ist hauptsächlich parallel zur Zentralachse angeordnet. Damit kann das Stell-Element aus derselben Richtung, wie das Verriegelung-Element, mit Hilfe von Instrumenten angetrieben werden. In der Axial-Öffnung wird das Stell-Element längenverstellbar geführt. Mit dem Begriff Axial-Öffnung sind, neben vollständig von Material umschlossene Öffnungen, auch Teil-Öffnungen oder Teil-Ausschnitte wie beispielsweise c-förmige Ausschnitte quer zur Zentralachse gemeint.

Quer zu der Axial-Öffnung und der Zentralachse ist eine zusätzliche Öffnung, eine Quer-Öffnung, vorgesehen. Die Quer-Öffnung stellt eine Verbindung zwischen Axial-Öffnung und Durchgangs-Öffnung des Gabelkopfs her. In dieser Quer-Öffnung ist das Fixier-Element rotierbar um die Quer-Öffnungs-Achse beweglich geführt. Mit Einleitung eines Torsionsmoments um die Quer-Öffnungs-Achse klemmt das Fixier-Element den Kopfbereich des Knochenankers winkelstabil im Gabelkopf. Die Quer-Öffnung bzw. die Achse der Quer-Öffnung ist dabei in etwa orthogonal zur Zentralachse und auch orthogonal zur Axial-Öffnung angeordnet.

Die temporäre Klemmung bzw. die Kompressionskraft wird vorzugsweise durch ein Stell-Element, welches in der Axial-Öffnung geführt ist durch Verstellung des Stell-Elements erzeugt. Die Kompressionskraft des Stell-Elements wird dabei auf das Fixier-Element übertragen bzw. umgelenkt, so dass sich das Fixier-Element um die Quer-Öffnungs-Achse dreht und dadurch ein Torsionsmoment generiert wird, was zur temporären Klemmung des Knochenanker-Kopfbereichs im Gabelkopf führt.

Der temporäre Klemmwirkung kann signifikant erhöht werden, wenn ein Hebel am Fixier-Element vorgesehen ist. Eine Betätigung des Hebels erzwingt eine Rotation des Fixier-Elements und löst damit die temporäre Klemmung aus. Für den Aufbau ist es notwendig, dass das Fixier-Element seitlich über die Wandungen des Gabelkopfs hinausragt, so dass der Hebel fest mit dem Fixier-Element verbunden ist. In einer alternativen Ausführungsform kann das Stell-Element nun am Hebelende angreifen, um den Hebel permanent und ohne zusätzliche Instrumente mit einer Kraft zu beaufschlagen.

Alternativ ist es auch denkbar, dass die temporäre Klemmung bzw. die Einleitung einer Kompressionskraft über das Fixier-Element selbst erfolgen kann. Hierfür wäre es beispielsweise erforderlich, dass das Fixier-Element und die Quer-Öffnung mit Hilfe eines Gewindeabschnitts miteinander im Eingriff stehen und so eine Längenverstellung ermöglicht wird. Durch diese Längenverstellung verschiebt sich das Fixier-Element entlang der Quer-Öffnungs-Achse. Ist eine Durchmesseränderung entlang des Fixier-Elements vorgesehen, kann hier ein Kraft-Schluss zwischen dem Fixier-Element und dem Kopfbereich des Knochenankers erreicht werden.

Optimalerweise stellt der Gabelkopf mindestens eine seitliche Aussackung oder Materialaufdickung bereit, um darin die Axial-Öffnung, Quer-Öffnung und die zur Betätigung notwendigen Elemente (Stell- und Fixier-Element) aufzunehmen. In der alternativen Ausgestaltungsform mit zusätzlichem Hebel ist es vorteilhaft, wenn an jedem der beiden Gabelkopf-Schenkel (11, 12) eine Aussackung vorgesehen ist.

In einer bevorzugten Ausführungsform verfügt der Gabelkopf über ein Druckstück, welches einen nach distal gerichteten Kontakt-Bereich zum Knochenanker-Kopfbereich und ein nach proximal gerichtetes Stablager ausweist. Im Bereich des Knochenanker-Kopfbereichs ist eine seitliche Öffnung oder ein teilweiser Ausschnitt vorgesehen, worin das Fixier-Element frei beweglich angeordnet ist. Dadurch ist eine Aktivierung des Fixier-Elements ohne die Belastung des Druckstücks ermöglicht.

In einer bevorzugten Ausführungsform kann der Gabelkopf von distal kommend mit Knochenankern montiert werden. Durch diese vorteilhafte Anordnung der Komponenten können die Knochenanker durch Aufsetzen bzw. Aufpressen relativ einfach mit dem Gabelkopf montiert werden. Durch ein Hilfsmittel, wie beispielsweise einem Löse-Instrument, ist der Knochenanker auch wieder entfernbar. Damit kann die erfindungsgemäße Osteosynthesevorichtung modular vom Anwender konfiguriert und im Operationssaal zu einem späteren Zeitpunkt als der der Fertigung, zusammengesetzt werden. Dadurch ist es beispielsweise möglich, dass zuerst der Knochenanker einzeln in den Knochen verankert oder geschraubt wird, und dann im Anschluss der Gabelkopf am bereits implantierten Knochenanker befestigt wird. Dies hat den großen Vorteil, dass der Chirurg nach der Implantation des Knochenankers deutlich mehr im Platz und eine bessere Sicht im OP-Feld im Vergleich zu den sonst vollständig implantierten Pedikelschrauben hat.

Vorteil ist, dass einerseits größere Knochenanker, d.h. Knochenanker mit einem größeren Außendurchmesser, als der distale Innendurchmesser des Gabelkopfes montiert werden können. Andererseits kann das Knochenanker-Portfolio minimalisiert werden, da der Anwender Gabelkopf und Knochenanker während der Operation miteinander kombinieren kann, anstatt auf ein vorgefertigtes übergroßes Portfolio zurückzugreifen. Ein solches Portfolio muss beim Anwender vorrätig sein und somit ist deutlich mehr Kapital gebunden, als es die erfindungsgemäße modulare Version erfordern würde.

Für eine modulare Gestaltung der Osteosynthesevorrichtung ist es vorteilhaft, wenn das Druckstück in distaler Richtung offene Schlitze aufweist und dadurch wenigstens drei federelastische Arme am Kopfaufnahmebereich ausgebildet sind. Die federelastische Arme können nach radial außen auslenken und somit den Knochenanker-Kopfbereich umschließen. Dadurch kann ein Knochenanker von distaler Richtung kommend in das Druckstück eingeklippt werden. Das Druckstück formt am distalen Ende der Außenseite wenigstens abschnittsweise einen Konus. Im Gabelkopf auf Höhe des Kugelaufnahmebereichs ist wenigstens ein Innen-Konus-Abschnitt definiert, welche kongruent zum Konus des Druckstücks und bei Betätigung des Verriegelung-Elements zur winkelstabilen Klemmung des Knochenanker-Kopfbereichs mit dem Gabelkopf führt. Auch hier ist es wesentlich, dass das Druckstück eine Durchführungsöffnung für das Fixier-Element vorsieht, damit das Druckstück bei aktivierter temporärer Klemmung nicht belastet wird.

Am proximalen Gabelkopfbereich ist eine umlaufene Nut mit einem hakenähnlichen Profil vorgesehen, die einen Hintergriff für ein Instrument bereitstellt. Stellvertretend sind andersartig gestaltete Nutprofile oder auch andere Haltemerkmale wie z.B. Öffnungen denkbar, die einen Hintergriff für ein Instrument bereitstellen.

Am proximalen Ende des Gabelkopfs können sich weitere und lösbare Abschnitte mit einem Gewindebereich befinden, die eine Reposition des Verbindungsstabs erlauben. Es ist auch denkbar, dass eine durch zwei längere Schenkel ausgebildeter hülsenähnlicher Zugang vorgesehen ist, wie es für den minimal-invasiven Zugang zum Einsatz kommt. Dabei können die lösbaren Schenkelverlängerungen optional am proximalen Ende miteinander verbunden sein. Mit lösbarer Verbindung sind beispielsweise Sollbruchstellen gemeint, die dazu geeignet sind, die Verlängerungen nach final fixiertem Verbindungsstab zu entfernen.

Als Material eignen sich alle metallischen Legierungen, die als orthopädischer Implantatwerkstoff bekannt und akzeptiert sind. Dazu gehören beispielsweise Titan-, Cobalt-Chrom und Edelstahllegierungen. Sollte die herkömmliche Herstellung des Gabelkopfes und des Verriegelungsrings nicht oder nur unter höchsten technologischen Aufwand möglich sein, stellt die additive Fertigung das Mittel der Wahl dar. Additive Fertigungen von metallischen Legierungen, oder auch 3D-Druck genannt, greifen auf das Laser- oder Elektronenstrahl-Schmelzverfahren zurück.

Weitere Merkmale, Vorteile und Einzelheiten der Erfindung ergeben sich aus den beigefügten Patentansprüchen, der zeichnerischen Darstellungen und nachfolgenden Beschreibung bevorzugter Ausführungsformen der erfindungsgemäßen Osteosynthesevorrichtung.

### Kurze Beschreibung der Zeichnungen zeigen

Fig. 1 eine Schrägansicht der erfindungsgemäßen Osteosynthesevorrichtung mit der Vorortung der Raumbeziehungen,
Fig. 2 die fertig implantierte Osteosynthesevorrichtung in einer Schrägansicht,
Fig. 3a eine Schrägansicht der erfindungsgemäßen Osteosynthesevorrichtung, und
Fig. 3b eine Explosionsdarstellung der erfindungsgemäßen Osteosynthesevorrichtung bestehend aus einem Gabelkopf, einem Fixier-Element, Stell-Element, Knochenanker und Druckstück,
Fig. 4 eine Seitenansicht der montierten erfindungsgemäßen Osteosynthesevorrichtung mit dazu gehöriger Schnittdarstellung,
Fig. 5a, b zwei unterschiedliche Stellungen S1, S2 des Stell-Elements
Fig. 6 zeigt eine Explosionsdarstellung einer alternativen Ausgestaltungsform, bei der das Fixier-Element einen Hebel besitzt.
Fig. 7 stellt eine Seitenansicht der montierten erfindungsgemäßen Osteosynthesevorrichtung aus Fig. 6 mit entsprechender Schnittdarstellung vor.

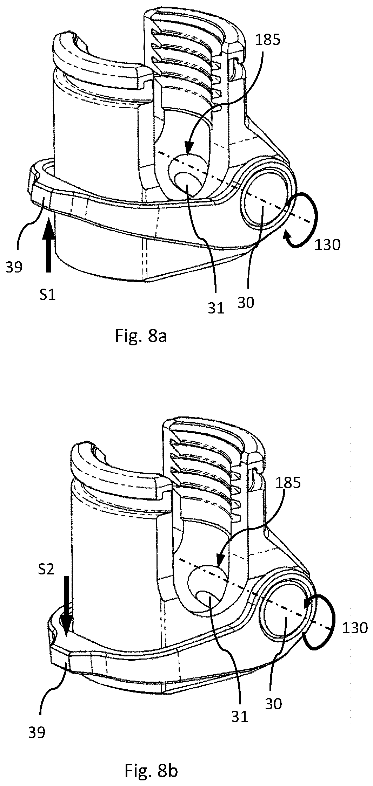

Fig. 8a und 8b illustrieren die Funktionsweise des Hebels.
Fig. 9a, b zeigen eine alternative Ausgestaltungsform, bei der Hebel mit einem Stell-Element betätigt wird.
Fig. 10 stellt eine Seitenansicht mit entsprechender Schnittdarstellung der Ausführungsform aus Fig. 9a und 9b dar.

### Beschreibung der bevorzugten Ausführungsformen

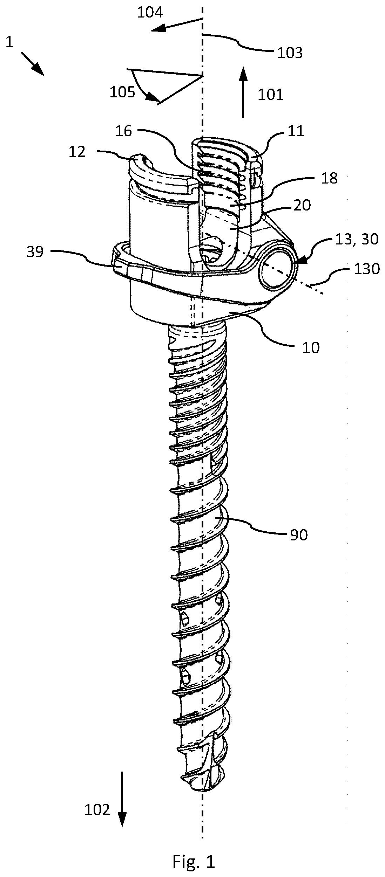

Beschrieben wird eine Osteosynthesevorrichtung (1), zur Behandlung der Wirbelsäule, wobei mehr als eine Osteosynthesevorrichtung (1) eingesetzt wird, um ein oder mehr Wirbel mit Hilfe von Verbindungsstäben (50) miteinander zu verbinden und somit die Wirbelsäule zu stabilisieren. Für die Osteosynthesevorrichtung (1), insbesondere für den Gabelkopf (10) sind raumzuweisende Koordinatenreferenzen definiert, wie zum Beispiel die proximale Richtung (101), die distale Richtung (102), die sich entlang einer Zentralachse (103) ausdehnen. Von der Zentralachse (103) nach außen abgehend definiert die radiale Ausbreitung (104) und die umfängliche Ausbreitung (105) ist durch einen konstanten Radius und einen variablen Umfangswinkel definiert (Fig. 1).

Fig. 1 zeigt eine Ausführungsform der erfindungsgemäßen Osteosynthesevorrichtung (1), zur Behandlung der Wirbelsäule, bestehend aus einem in einer Seitenansicht u-förmigen Gabelkopf (10), welcher eine Durchgangsöffnung (18) besitzt und der Gabelkopf (10) in proximaler Richtung (101) zwei Gabelschenkel (11, 12) mit einem innenliegenden Gewinde (16) aufweist, und darin ein Verbindungsstab (50) aufgenommen werden kann, und im Gabelkopf (10) in distaler Richtung (102) in der Durchgangsöffnung (18) ein Kugelkopfaufnahmebereich (19) vorgesehen ist und darin ein Knochenanker (90) schwenkbar gelagert ist. Im Gabelkopf (10) ist eine Quer-Öffnung (13) vorgesehen, welche mit der Durchgangsöffnung des Gabelkopfs (18) kommuniziert. In dieser Quer-Öffnung (13) ist ein Fixier-Element (30) drehbar um eine Achse (130) beweglich geführt. Mit Einleitung eines Torsionsmoments um die Quer-Öffnungs-Achse (130) klemmt das Fixier-Element (30) den Kopfbereich (91) des Knochenankers (90) winkelstabil im Gabelkopf (10). Mit Entlastung des Torsionsmoments am Fixier-Element (30) wird der Kopfbereich (91) des Knochenankers (90) im Gabelkopf (10) wieder beweglich.

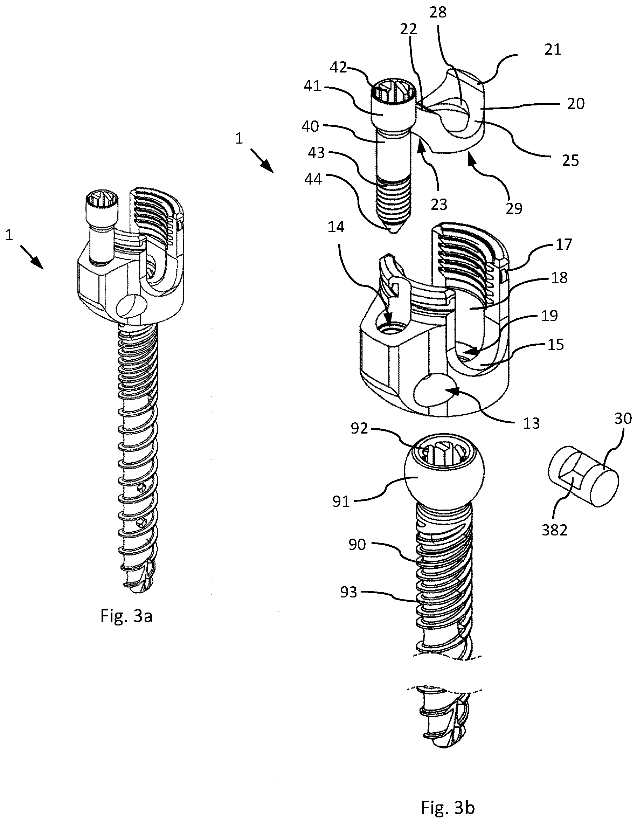

Fig. 1, 3a und 3b illustrieren auch, dass der Gabelkopf (10) der Osteosynthesevorrichtung (1) über ein Druckstück (20) verfügt und dass das Druckstück eine Durchgangsöffnung (28), einen nach distal gerichteten Kontakt-Bereich zum Knochenanker-Kopfbereich (91), ein nach proximal gerichtetes Stablager (25), welches durch zwei Schenkel (21, 22) abgegrenzt ist und im Bereich des Knochenanker-Kopfbereichs (91) eine seitliche Öffnung oder teilweisen Ausschnitt (23) aufweist, worin das Fixier-Element (30) frei beweglich angeordnet ist. Wird ein Verbindungsstab (50) in die u-förmige Gabelöffnung (15) eingelegt, steht der Verbindungsstab (50) mit dem Stablager des Druckstücks (25) in direktem Kontakt. Wird nun das Verriegelungs-Element (60) mit dem Gabelkopf (10) fixiert, wird eine Kompressionskraft vom Verriegelungs-Element (60) auf den Verbindungsstab (50), und von diesem auf das Druckstück (20, 25) und vom Druckstück (20) auf den Knochenanker-Kopfbereich (91), und der Knochenanker (90) gegen den distalen Kugelsitzbereich (19) übertragen. Diese eingeleitete Kompressionskraft führt dann zur Klemmung der Polyaxialität.

Der Knochenanker (90) weist vorzugsweise einen Kopfbereich (91) mit einer darin befindlichen Werkzeugansatzstelle (92) auf und besitzt in distaler Richtung (102) ein Knochengewinde (93).

Fig. 3b zeigt einen bevorzugten Aufbau des Fixier-Elements (30). Hier dargestellt ist, dass das Fixier-Element (30) vorzugsweise in der hauptsächlichen Gesamtform als Rundstab oder Stift gestaltet und in einer konzentrischen Quer-Öffnung (13) rotatorisch gelagert ist. Vorstellbar sind auch alternative aber hier nicht gezeigte Ausführungen des Fixier-Elements (20), wie beispielsweise, dass beim Fixier-Element in einer Schnittdarstellung quer zur Quer-Öffnungs-Achse (130) wenigstens ein Abschnitt als Dreieck, Viereck oder Vieleck, oder auch als Drei- oder Vieleck mit Übergangsrundungen ausgestaltet ist, oder als Vollrund mit seitlichen Abflachungen vorgesehen ist, die dazu dienen, dass eine Rotation des Fixier-Elements (30) eine Klemmung am Knochenanker-Kopfbereich (91) hervorruft. Denkbar ist auch, dass das Fixier-Element (30) entlang der Quer-Öffnungs-Achse (130) seine Geometrie ändert, um Eingriffsmerkmale für Verliersicherungs-Elemente bereitzustellen.

In der in Fig. 3b gezeigten Ausführungsform verfügt der Gabelkopf (10) über eine Axial-Öffnung (14), worin ein Stell-Element (40) in der Axial-Öffnung (14) geführt ist und durch Verstellung des Stell-Elements (40) eine Kompressionskraft erzeugt wird, welche direkt auf das Fixier-Element (30) übertragen wird. Dadurch wird ein Torsionsmoment am Fixier-Element (30) um die Quer-Öffnungs-Achse (130) erzeugt, welches zur temporären Klemmung des Knochenanker-Kopfbereichs (91) im Gabelkopf (10) führt. Vorteilhaft ist es, wenn die Axial-Öffnung (14) für das Stell-Element (40) parallel aber in einem Abstand zur Zentralachse (103) angeordnet ist. Die Osteosynthesevorrichtung (1) besitzt vorzugsweise ein stiftförmiges Stell-Element (40), welches in proximaler Richtung (101) am Kopf (41) eine Werkzeugansatzstelle aufweist (42) (Fig. 3a, b). Das Stell-Element (40) ist lösbar gestaltet und kann ggf. auch entfernt werden. Mit entferntem Stell-Element (40) und nicht temporär geklemmten Fixier-Element (30) verhält sich diese Osteosynthesevorrichtung (1) wie eine polyaxiale Pedikelschraube.

Fig. 3b zeigt auch, wenn eine Axial-Öffnung (14) in einem Gabelkopf-Schenkel (11, 12) vorgesehen ist, dass es vorteilhaft ist, dass der Gabelkopf (10) eine seitliche Materialwulst besitzt, welche in einen der Schenkel (11 oder 12) übergeht, damit die Quer-Öffnung (13) als auch die Axial-Öffnung (14) überhaupt herstellbar sind.

Der Fig. 3b ist ebenfalls zu entnehmen, dass der Gabelkopf (10) Vorsprünge, Öffnungen, Nuten, Stege, Profile oder sonstige Merkmale (17) die zum Er-, Ein- oder Hintergreifen mit einem Instrument geeignet sind, besitzt. Wenn das Stell-Element (40) Teil eines Instruments ist kann dieses Instrumenten-Anbindungsmerkmal (17) dazu dienen eine Zugkraft am Gabelkopf (10) einzuleiten, welche als Antagonist für das Einleiten einer Kompressionskraft über das Stell-Element (40) wirkt.

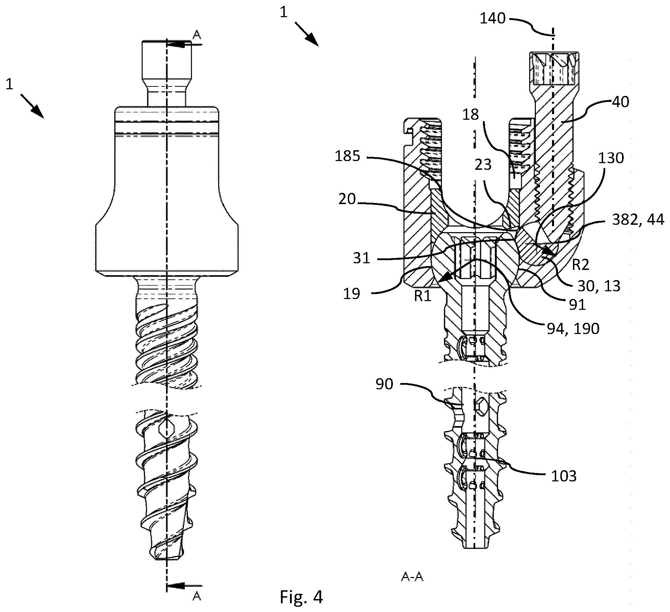

Fig. 4 zeigt die erfindungsgemäße Osteosynthesevorrichtung (1) in einer Seitenansicht und im Schnitt. Erkennbar ist, dass das Zentrum des innenliegenden Gewindes (16) und das Zentrum des Kugelkopfaufnahmebereichs (190) eine Zentralachse (103) definieren, und sich diese Zentralachse (103) nicht mit der Quer-Öffnungs-Achse (130) schneidet. Die Quer-Öffnungs-Achse (130) ist in etwa orthogonal und in einem Abstand zur Zentralachse (103) angeordnet. Das Fixier-Element (30) ragt mindestens abschnittsweise in die Durchgangsöffnung (18) des Gabelkopfs (10) hinein (185) und liegt am Kugelkopfbereich (91) des Knochenankers (90) an. Die Quer-Öffnung (13) kommuniziert mit der Durchgangsöffnung des Gabelkopfs (18) über einen Wandungsausschnitt (185). Durch diesen Wandungsausschnitt (185) hat das Fixier-Element (30) direkten Zugang zum Knochenanker-Kopfbereich (91). Optimalerweise befindet sich der Kontaktbereich des Fixier-Elements (30) genau an dieser Stelle des Wandungsausschnitts (185).

Des Weiteren ist es vorteilhaft, wenn die Kontaktstelle (31) zur Einleitung der temporären Klemmung in proximaler Richtung (101) oberhalb des Äquators (94) des Knochenanker-Kopfbereichs (91) oder des Zentrums (190) des Kugelkopfaufnahmebereichs im Gabelkopf (19) liegt. Dadurch wird erreicht, dass bei eingeleiteter Kompressionskraft durch das Fixier-Element (30) der Knochenanker-Kopfbereich (91) nicht nur in die gegenüberliegende Richtung bzw. an die gegenüberliegende Innenwandung des Gabelkopfs (18) gedrückt wird, sondern auch anteilsweise in den Kugelsitz (19) gezwungen wird. Somit wird der Knochenanker (90) im Gabelkopf (10) im Kugelsitz (19) unter Krafteinwirkung zentriert. Dadurch wird die Kompressionskraft auf den Mittelpunkt (190) der kugelähnlichen Knochenanker-Kopfbereich (91) gerichtet, so dass auch bei verschwenkten Knochenanker (90) ein ähnlich-großer Kontaktbereich zwischen Fixier-Element und Knochenanker-Kopfbereich (91) vorliegt.

Der für die temporäre Klemmung verantwortliche nach radial innen gerichteten Kontakt-Bereich (31) des Fixier-Elements (30), liegt unmittelbar am Kopfbereich (91) des Knochenankers (90) an. Vorteilhaft ist es, wenn dieser Kontakt-Bereich (31) in einer Seitenansicht wenigstens abschnittsweise konkav ausgeformt ist oder wenigstens einen Abschnitt der Außenfläche des Knochenanker-Kopfbereichs (91) approximiert. Für eine optimierte Klemmwirkung ist es vorteilhaft, wenn der nach radial innen gerichtete Kontakt-Bereich (31) des Fixier-Elements (30) eine erhöhte Rauigkeit, Kerben oder Zähne aufweist (Fig. 4). Vorteilhaft für die Herstellung ist es, wenn der nach radial innen gerichtete Kontaktbereich als Ausschnitt des sonst stift-ähnlichen Fixier-Elements ausgestaltet ist. Damit das Stift-ähnliche Fixier-Element (30) eine Klemmwirkung ausüben kann, ist es vorteilhaft, wenn der Kopfbereich (91) des Knochenankers (90) durch einen Radius R1 und das Fixier-Element (30) an seiner dicksten Stelle durch einen Radius R2 definiert ist, und der kürzeste Abstand zwischen der Zentralachse (103) und der Quer-Öffnungs-Achse (130) kleiner ist als die Summe der Radien R1 und R2. Dadurch liegt eine rechnerische Materialüberdeckung des Knochenanker-Kopfbereichs mit dem Fixier-Element vor. Der im Fixier-Element ausgeschnittene Kontaktbereich (31) stellt den entsprechenden Freiraum zum Knochenanker-Kopfbereich. Eine Verdrehung des Fixier-Elements (30) um die Quer-Öffnungs-Achse (130) erzwingt eine Materialüberdeckung, was dann zur Klemmung des Knochenanker-Kopfbereichs (91) im Kugelsitz des Gabelkopf (19) resultiert. Alternativ kann dies auch über einen abschnittsweisen Exzenter als Teil des Fixier-Elements (30) erreicht werden.

In Fig. 4 zeigt auch dass ein Stell-Element (40) vorgesehen ist. Durch Verstellung des Stell-Elements (40) kann eine Kompressionskraft erzeugt werden, welche auf das Fixier-Element (30) übertragen bzw. umgelenkt wird, und dadurch wird ein Torsionsmoment um die Quer-Öffnungs-Achse (130) erzeugt, welches zur temporären Klemmung des Knochenanker-Kopfbereichs (91) im Gabelkopf (10) führt.

Dabei wird das Stell-Element (40) in der Axial-Öffnung (14) längenverstellt geführt. Ist das Stell-Element (40) Teil des Implantats ist es vorteilhaft, wenn die Axial-Öffnung (14) für das Stell-Element (40) wenigstens abschnittsweise ein Innengewinde besitzt. Damit im Eingriff stehend ist es erforderlich, dass das Stell-Element (40) selbst auch wenigstens abschnittsweise ein Gewinde (43) besitzt. Dadurch ist es möglich, dass bei eingeschraubten bzw. angezogenem Stell-Element (40) die eingeleitete Kompressionskraft aufrechterhalten bleibt. Ist das Stell-Element (40) Teil eines Instruments muss das Stell-Element (40) selbst kein Gewinde aufweisen, da die einzuleitende Kompressionskraft durch das Instrument generiert wird.

Für die Umlenkung der Kompressionskraft vom Stell-Element (40) auf das Fixier-Element (30) ist es vorteilhaft, wenn das Fixier-Element (30) wenigstens eine Kontaktfläche (382) aufweist, welche in direktem Kontakt mit einem distalen Kontakt-Bereich (44) des Stell-Elements (40) steht und dieser Kontakt (382, 44) so ausgestaltet ist, dass eine Kompressionskraft entlang der Stell-Element-Achse (140) in eine Rotation um der Quer-Öffnungs-Achse (130) umgeleitet wird. Dabei wirkt die Kontaktfläche (382) des Fixier-Elements (30) als integrierter Hebel (Fig. 4). Deshalb ist es wichtig, dass die Achse des Stell-Elements (140) in einem Abstand zur Quer-Öffnungs-Achse (130) steht. Des Weiteren ist es in dieser Ausführungsform so vorgesehen, dass der distale Kontakt-Bereich (44) des Stell-Elements (40) wenigstens abschnittsweise konvex ausgestaltet ist, um eine tangentiale Führung bzw. gleichbleibenden Kontakt bei eingeleiteter Rotation des Fixier-Elements sicherzustellen.

In Fig. 4 ist auch das wesentliche charakteristische Merkmal zu erkennen, nämlich dass bei temporärer Klemmung des Knochenanker-Kopfbereichs (91) alleinig durch das Fixier-Element (30) das Druckstück (20) stets unbelastet ist. Erst durch Einlegen und Fixieren eines Verbindungsstabs (50, 60) wird das Druckstück mit einer Kraft beaufschlagt. Dadurch kann der Gabelkopf und das Druckstück eine höhere mechanische Belastung aufnehmen und das Druckstück kann mit deutlich weniger Material als vergleichbare Konzepte vorgesehen werden, was sich wiederrum positiv auf die Bauhöhe der Osteosynthesevorrichtung (1) auswirkt. Bei Osteosynthesevorrichtungen (1) für die Wirbelsäule ist eine kleinstmögliche Bauhöhe wichtig, um sich bestmöglich den anatomischen Gegebenheiten des Patienten anzupassen.

Die Osteosynthesevorrichtung (1) ist so ausgestaltet, dass ein Verbindungsstab (50) einlegbar und mit einem Verriegelungs-Element (60) am Gabelkopf (10) fixierbar ist, und dadurch eine winkelstabile Klemmung zwischen Knochenanker-Kopfbereich (91) und Gabelkopf (10) erzielt wird, und die winkelstabile Klemmung mit Hilfe des Verriegelung-Elements (60) und die winkelstabile Klemmung mit Hilfe des Fixier-Elements (30) unabhängig voneinander aktivierbar und auch miteinander kombinierbar sind.

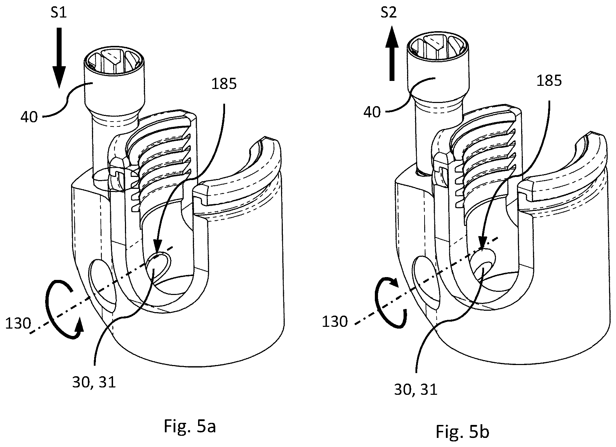

Fig. 5a und 5b ist zu entnehmen, dass das Fixier-Element (30) um die Quer-Öffnungs-Achse (130) eine Stellung S1 einnehmen kann, bei der eine Kompressionskraft auf den Knochenanker (90) übertragen wird, so dass Knochenanker (90) winkelstabil im Kugelsitz (19) gehalten wird und das Fixier-Element (30) eine zweite Stellung S2 einnehmen kann, bei der der Knochenanker (90) beweglich im Kugelsitz (19) gehalten wird. Eingestellt werden können diese Stellungen des Fixier-Elements (30) durch eine Verstellung des Stell-Elements (40). Dies ist durch die unterschiedliche Rotationsstellungen des Fixier-Elements (30) im Wandungsausschnitt (185) zu erkennen.

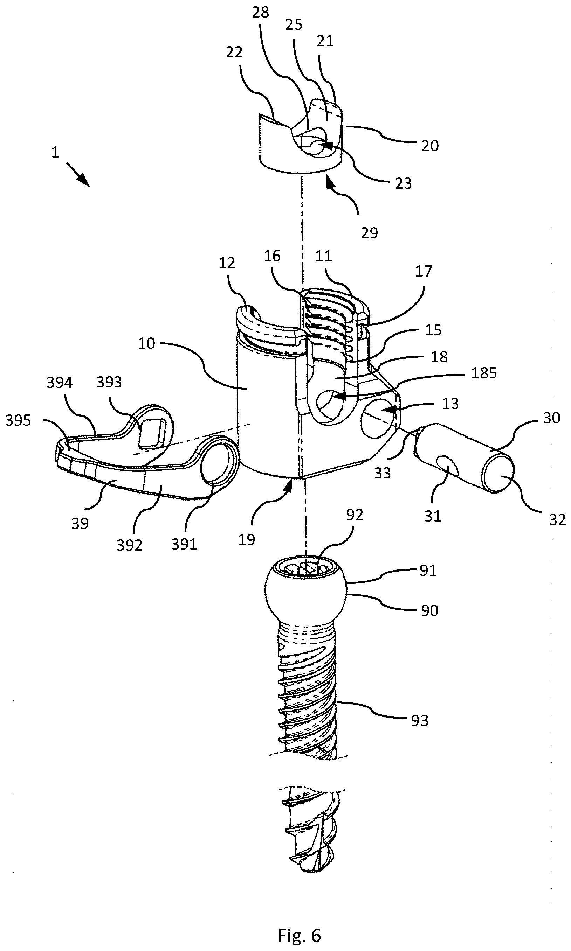

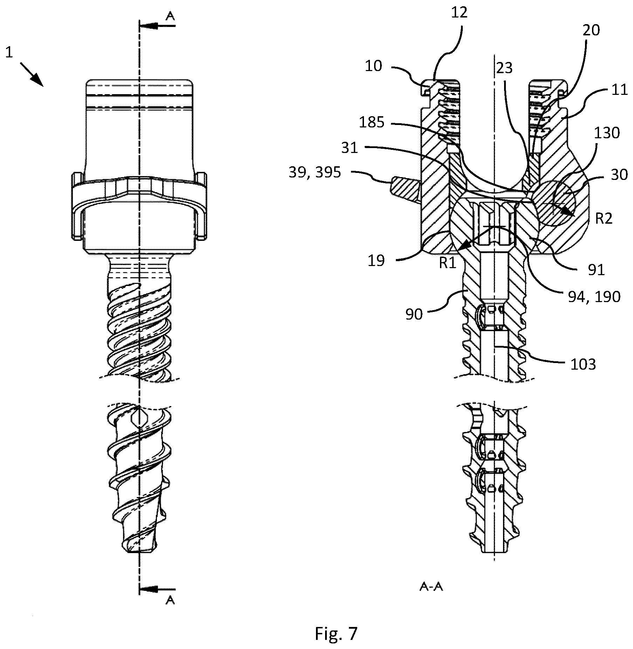

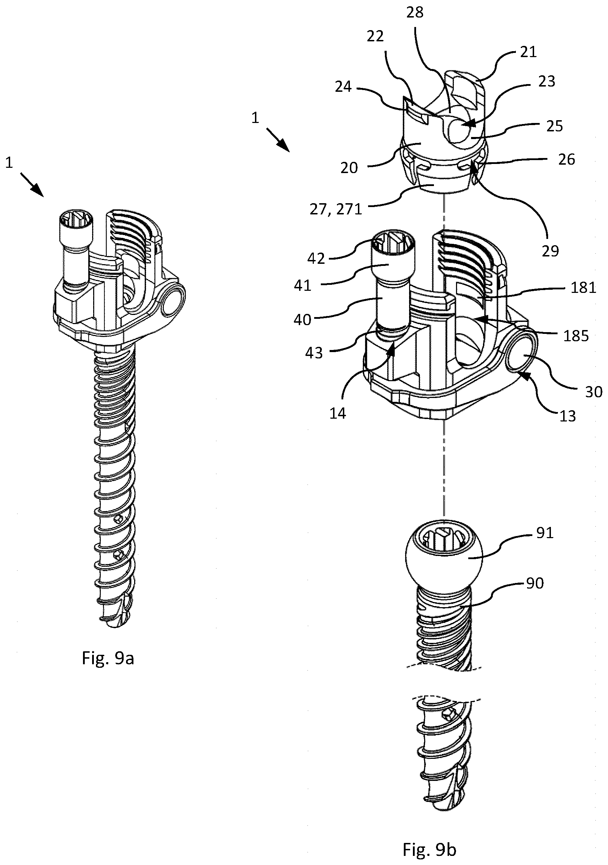

In Fig. 6 und Fig. 7 ist eine alternative Ausgestaltungsform der Osteosynthesevorrichtung zu erkennen, bei der die Klemmkraft durch einen zusätzlichen Hebel (39) erhöht werden kann. Dabei ist es vorteilhaft, wenn sich das Fixier-Element (30) über wenigstens eine seitliche Wandung des Gabelkopfs (10) hinaus erstreckt (32, 33) und daran der Hebel (39) befestigt ist. Eine Betätigung des Hebels (39) bewirkt eine Rotation des Fixier-Elements (30) und erzwingt damit die temporäre Klemmung des Knochenanker-Kopfbereichs (91) im Gabelkopf (10). Die Auslenkung des Hebels wird vorzugsweise an einem Bereich (395) eingeleitet, welcher einen größtmöglichen Abstand zur Quer-Öffnung (13) aufweist, um die Hebelwirkung, d.h. die Kraftverstärkung, zu maximieren. Optimalerweise besitzt das Fixier-Element (30) zwei Enden (32, 33) die über die seitliche Wandung des Gabelkopfs hinausragen. Der Hebel besitzt vorzugsweise zwei Schenkel (392, 394), die an den Enden des Fixier-Elements (32, 33) mit dem Hebel verbunden oder miteinander gefügt sind (391, 393). Vorteilhaft ist es, wenn die Fügestellen zusätzlich einen Formschluss (393) enthalten, damit eine höhere Übertragung von Belastungen möglich ist. Optimalerweise ist der Hebel außerhalb des Gabelkopfs (10) angeordnet, so dass er mit geeigneten Stell-Mitteln wie dem Stell-Element (40, Fig. 9a, 9b) oder einem hier nicht gezeigtem Instrument betätigt werden kann. Die Betätigung bzw. das Wechseln zwischen zwei Stell-Positionen mit der sich einstellenden Klemmwirkung ist in den Figuren 9a und 9b dargestellt.

In Fig. 9a und 9b ist eine weitere bevorzugte Ausführungsform der erfindungsgemäßen Osteosynthesevorrichtung (1) gezeigt. Hier weist das Druckstück (20) in distaler Richtung (102) offene Schlitze (26) auf. Dadurch sind wenigstens drei federelastische Arme (27) am Kopfaufnahmebereich (29) ausgebildet, wobei die federelastische Arme (27) an ihrer Außenseite wenigstens abschnittsweise einen Konus (271) beschreiben, und der Knochenanker (90) von distaler Richtung (102) kommend in den Gabelkopf (10) gesteckt werden kann. Dadurch können Knochenanker (90) mit größerem Außendurchmesser mit dem Gabelkopf (10) montiert werden.

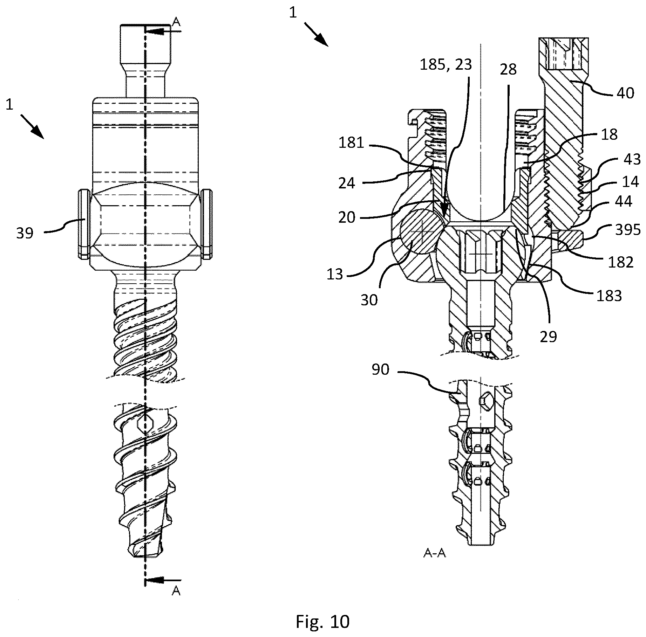

Damit das Druckstück innerhalb des Gabelkopfs (10) optimal geklemmt werden kann, ist im Kugelaufnahmebereich (19) wenigstens ein Innen-Konus-Abschnitt (183) definiert, welcher kongruent zum Konus (271) des Druckstücks (20) vorgesehen ist und bei Betätigung des Verriegelung-Elements (60) zur winkelstabilen Klemmung des Knochenanker-Kopfbereichs (91) mit dem Gabelkopf (10) führt (Fig. 9a, b und Fig. 10). Auch hier ist es erforderlich, dass das Druckstück eine seitliche Öffnung oder einen wenigstens teilweisen Ausschnitt (23) besitzt durch den das Fixier-Element (30) geführt ist und darin sich bewegen kann. Dadurch wird erreicht, dass das Druckstück (20) bei aktivierter temporärer Klemmung nicht belastet wird.

In Fig. 9a, 9b ebenfalls illustriert ist eine alternative Ausgestaltungsform bei welcher der Hebel (39) durch ein Stell-Element (40) betätigt werden kann. Dieser Aufbau ist dafür geeignet, dass die Klemmwirkung durch das Stell-Mittel (40) ohne Instrumente aufrechterhalten bleibt.

In Fig. 10 ist zu entnehmen, dass die Gabelkopf-Schenkel (11, 12) jeweils eine in proximaler Richtung (101) wirksame Abstützflache (181) mit einer Hinterschneidung bereitstellen, und das Druckstück (20) am proximalen Ende nach radial außen gerichtete Vorsprünge (24) besitzt, wobei die Vorsprünge (24) nach radial innen federelastisch gestaltet sind, so dass das Druckstück (20) aus proximaler Richtung kommend (101) in den Gabelkopf (10) gesteckt werden kann. Dabei verrasten die Vorsprünge des Druckstücks (24) mit den Abstützflachen (181) und das Druckstück (20) wird in proximaler Richtung (101) gesichert aber nicht kraftbeaufschlagt. In Fig. 10 ist auch zu erkennen, dass der Gabelkopf (10) in der Durchgangsöffnung (18) einen Bereich besitzt, welcher wenigstens abschnittsweise einen größeren Innendurchmesser (182) besitzt als der Kerndurchmesser des Gewindes (16). Dadurch ist es möglich, wenn das Druckstück noch nicht vollständig mit dem Gabelkopf (10) in der finalen Position verrastet ist, dass dem federelastischen Konus-Bereich (27) des Druckstücks im Gabelkopf (10) auf Höhe der Innendurchmesser-Erweiterung (182) entsprechender Platz zum Aufspreizen für die Montage mit dem Knochenanker-Kopfbereich (91) bereitgestellt wird.

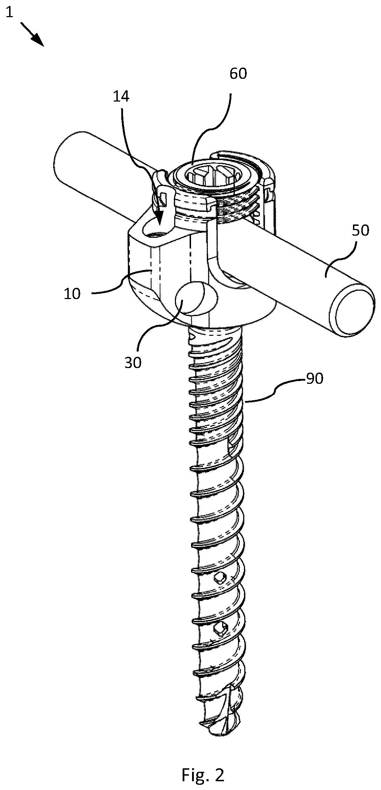

In Fig. 2 dargestellt ist, wenn das Stell-Element (40) innerhalb einer Axial-Öffnung (14) geführt ist und Teil der Osteosynthesevorrichtung (1) ist, und eine Sollbruchstelle (45) besitzt, dass nur ein Teil des Stell-Elements (40) nach dem finalen Verriegeln mit dem Verbindungsstab (50) und dem Verriegelungs-Element (60) im Patienten verbleibt. Ein gleiches Bild ergibt sich, wenn das Stell-Element (40) Teil eines Instruments ist und nach dem finalen Verriegeln mit dem Verbindungsstab (50) und dem Verriegelungs-Element (60) aus dem Patienten entfernt ist.

Alternativ kann das Stell-Element (40) auch komplett als Teil der Osteosynthesevorrichtung (1) vorgesehen sein. Nach dem finalen Verriegeln mit dem Verbindungsstab (50) und dem Verriegelungs-Element (60) verbleibt es im Patienten (nicht dargestellt). Vorteilhaft ist es dann, wenn das Stell-Element (40) nicht über das proximale Ende (101) des Gabelkopfes (10) hinausragt.

## Patentansprüche

1. Osteosynthesevorrichtung (1), zur Behandlung der Wirbelsäule, bestehend aus einem in einer Seitenansicht u-förmigen Gabelkopf (10), welcher eine Durchgangsöffnung (18) besitzt und der Gabelkopf (10) in proximaler Richtung (101) zwei Gabelschenkel (11, 12) mit einem innenliegenden Gewinde (16) aufweist, und darin ein Verbindungsstab (50) aufgenommen werden kann, und im Gabelkopf (10) in distaler Richtung (102) in der Durchgangsöffnung (18) ein Kugelkopfaufnahmebereich (19) vorgesehen ist und darin ein Knochenanker (90) schwenkbar gelagert ist, **dadurch gekennzeichnet, dass** am Gabelkopf (10) eine Quer-Öffnung (13) vorgesehen ist, welche mit der Durchgangsöffnung des Gabelkopfs (18) über einen Wandungsausschnitt (185) kommuniziert und in dieser Quer-Öffnung (13) ein Fixier-Element (30) um eine Quer-Öffnungs-Achse (130) drehbar gelagert ist und das Fixier-Element (30) mit Einleitung eines Torsionsmoments um die Quer-Öffnungs-Achse (130) den Kopfbereich (91) des Knochenankers (90) winkelstabil im Gabelkopf (10) klemmt, wobei das Zentrum des innenliegenden Gewindes (16) und das Zentrum des Kugelkopfaufnahmebereichs (190) eine Zentralachse (103) definieren, und sich diese Zentralachse (103) nicht mit der Quer-Öffnungs-Achse (130) schneidet.

2. Osteosynthesevorrichtung (1) nach Anspruch 1, **dadurch gekennzeichnet, dass** mit Entlastung des Torsionsmoments am Fixier-Element (30) der Kopfbereich (91) des Knochenankers (90) im Gabelkopf (10) beweglich wird.

3. Osteosynthesevorrichtung (1) nach einem der vorhergehenden Ansprüche, **dadurch gekennzeichnet, dass** die Quer-Öffnungs-Achse (130) in etwa orthogonal und in einem Abstand zur Zentralachse (103) angeordnet ist.

4. Osteosynthesevorrichtung (1) nach einem der vorhergehenden Ansprüche, **dadurch gekennzeichnet, dass** das Fixier-Element (30) mindestens abschnittsweise in die Durchgangsöffnung (18) des Gabelkopfs (10) hineinragt (185) und am Kugelkopfbereich (91) des Knochenankers (90) anliegt.

5. Osteosynthesevorrichtung (1) nach einem der vorhergehenden Ansprüche, **dadurch gekennzeichnet, dass** ein nach radial innen gerichteter Kontaktbereich (31) des Fixier-Elements (30) in einer Seitenansicht wenigstens einen Abschnitt der Außenfläche des Knochenanker-Kopfbereichs (91) approximiert.

6. Osteosynthesevorrichtung (1) nach einem der vorhergehenden Ansprüche, **dadurch gekennzeichnet, dass** ein nach radial innen gerichteter Kontaktbereich (31) des Fixier-Elements (30) eine erhöhte Rauigkeit, Kerben oder Zähne aufweist.

7. Osteosynthesevorrichtung (1) nach einem der vorhergehenden Ansprüche, **dadurch gekennzeichnet, dass** ein Kontaktbereich (31) zur Einleitung der temporären Klemmung in proximaler Richtung (101) oberhalb des Äquators (94) des Knochenanker-Kopfbereichs (91) liegt.

8. Osteosynthesevorrichtung (1) nach einem der vorhergehenden Ansprüche, **dadurch gekennzeichnet, dass** das Fixier-Element (30) im Wesentlichen stiftförmig ausgebildet ist.

9. Osteosynthesevorrichtung (1) nach einem der vorhergehenden Ansprüche, **dadurch gekennzeichnet, dass** der Kopfbereich (91) des Knochenankers (90) durch einen Radius R1 und das Fixier-Element (30) an seiner dicksten Stelle durch einen Radius R2 definiert ist, und der kürzeste Abstand zwischen der Zentralachse (103) und der Quer-Öffnungs-Achse (130) kleiner ist als die Summe der Radien R1 und R2.

10. Osteosynthesevorrichtung (1) nach einem der vorhergehenden Ansprüche, **dadurch gekennzeichnet, dass** sich das Fixier-Element (30) über wenigstens eine seitliche Wandung des Gabelkopfs (10) hinaus erstreckt (32, 33) und daran ein Hebel (39) befestigt (391, 393) ist und eine Betätigung des Hebels eine Rotation des Fixier-Elements (30) bewirkt.

11. Osteosynthesevorrichtung (1) nach einem der vorhergehenden Ansprüche, **dadurch gekennzeichnet, dass** der Gabelkopf (10) über eine Axial-Öffnung (14) verfügt, und ein Stell-Element (40) in der Axial-Öffnung (14) geführt ist und durch Verstellung des Stell-Elements (40) eine Kompressionskraft erzeugt wird, welche direkt auf das Fixier-Element (30) oder indirekt über einen Hebel (39) übertragen wird, und dadurch ein Torsionsmoment am Fixier-Element (30) um die Quer-Öffnungs-Achse (130) erzeugt wird, welches zur temporären Klemmung des Knochenanker-Kopfbereichs (91) im Gabelkopf (10) führt.

12. Osteosynthesevorrichtung (1) nach einem der vorhergehenden Ansprüche, **dadurch gekennzeichnet, dass** ein Verbindungsstab (50) einlegbar und mit einem Verriegelungs-Element (60) am Gabelkopf (10) fixierbar ist, und dadurch eine winkelstabile Klemmung zwischen Knochenanker-Kopfbereich (91) und Gabelkopf (10) erzielt wird, und die winkelstabile Klemmung mit Hilfe des Verriegelung-Elements (60) und die winkelstabile Klemmung mit Hilfe des Fixier-Elements (30) unabhängig voneinander aktivierbar und auch miteinander kombinierbar sind.

13. Osteosynthesevorrichtung (1) nach einem der vorhergehenden Ansprüche, **dadurch gekennzeichnet, dass** ein Stell-Element (40) nicht über das proximale Ende des Gabelkopfs (10) in proximaler Richtung (101) hinausragt.

14. Osteosynthesevorrichtung (1) nach einem der vorhergehenden Ansprüche, **dadurch gekennzeichnet, dass** das Fixier-Element (30) um die Quer-Öffnungs-Achse (130) eine Drehstellung S1 einnehmen kann, bei der eine Kompressionskraft auf den Knochenanker (90) übertragen wird, so dass der Knochenanker (90) winkelstabil im Kugelsitz (19) gehalten wird und das Fixier-Element (30) eine zweite Drehstellung S2 einnehmen kann, bei welcher der Knochenanker (90) beweglich im Kugelsitz (19) gehalten wird.

15. Osteosynthesevorrichtung (1) nach einem der vorhergehenden Ansprüche, **dadurch gekennzeichnet, dass** eine Axial-Öffnung (14) für ein Stell-Element (40) wenigstens abschnittsweise ein Innengewinde besitzt.

16. Osteosynthesevorrichtung (1) nach einem der vorhergehenden Ansprüche, **dadurch gekennzeichnet, dass** der Gabelkopf (10) über ein Druckstück (20) verfügt und das Druckstück eine Durchgangsöffnung (28), einen nach distal gerichteten Kontakt-Bereich zum Knochenanker-Kopfbereich (91), ein nach proximal gerichtetes Stablager (25) und im Bereich des Knochenanker-Kopfbereichs (29) (91) eine seitliche Öffnung oder teilweisen Ausschnitt (23) aufweist, worin das Fixier-Element (30) frei beweglich angeordnet ist.

## Claims

1. Osteosynthesis device (1) for treating the spine, comprising a fork head (10) which is U-shaped in a side view and which has a through opening (18) and the fork head (10) has two fork legs (11, 12) with an inner-lying thread (16) in the proximal direction (101), and a connecting rod (50) can be received therein, and a ball head receiving region (19) is provided in the fork head (10) in the distal direction (102) in the through opening (18), and a bone anchor (90) is pivotably mounted therein, **characterized in that** a transverse opening (13) is provided on the fork head (10), which communicates with the through opening of the fork head (18) via a wall cutout (185), and a fixing element (30) is mounted rotatably about a transverse opening axis (130) in this transverse opening (13), and the fixing element (30) clamps the head region (91) of the bone anchor (90) in an angularly stable manner in the fork head (10) with induction of a torsional moment about the transverse opening axis (130), wherein the centre of the inner-lying thread (16) and the centre of the ball head receiving region (190) define a central axis (103), and this central axis (103) does not intersect with the transverse opening axis (130).

2. Osteosynthesis device (1) according to claim 1, **characterized in that** the head region (91) of the bone anchor (90) in the fork head (10) becomes movable when the torsional moment at the fixing element (30) is unloaded.

3. Osteosynthesis device (1) according to one of the preceding claims, **characterized in that** the transverse opening axis (130) is arranged approximately orthogonally and at a distance from the central axis (103).

4. Osteosynthesis device (1) according to one of the preceding claims, **characterized in that** the fixing element (30) protrudes (185) at least in sections into the through opening (18) of the fork head (10) and abuts against the ball head region (91) of the bone anchor (90) .

5. Osteosynthesis device (1) according to one of the preceding claims, **characterized in that** a radially inwardly directed contact region (31) of the fixing element (30) approximates, in a side view, at least a portion of the outer surface of the bone anchor head region (91).

6. Osteosynthesis device (1) according to one of the preceding claims, **characterized in that** a radially inwardly directed contact region (31) of the fixing element (30) has an increased roughness, notches or teeth.

7. Osteosynthesis device (1) according to one of the preceding claims, **characterized in that** a contact region (31) for inducing the temporary clamping in the proximal direction (101) lies above the equator (94) of the bone anchor head region (91).

8. Osteosynthesis device (1) according to one of the preceding claims, **characterized in that** the fixing element (30) is substantially pin-shaped.

9. Osteosynthesis device (1) according to one of the preceding claims, **characterized in that** the head region (91) of the bone anchor (90) is defined by a radius R1 and the fixing element (30) is defined at its thickest point by a radius R2, and the shortest distance between the central axis (103) and the transverse opening axis (130) is smaller than the sum of the radii R1 and R2.

10. Osteosynthesis device (1) according to one of the preceding claims, **characterized in that** the fixing element (30) extends beyond(32, 33) at least one lateral wall of the fork head (10) and a lever (39) is attached thereto (391, 393), and an actuation of the lever causes the fixing element (30) to rotate.

11. Osteosynthesis device (1) according to one of the preceding claims, **characterized in that** the fork head (10) has an axial opening (14), and a positioning element (40) is guided in the axial opening (14), and a compression force is generated by adjusting the positioning element (40), which is transmitted directly on the fixing element (30) or indirectly via a lever (39), and a torsional moment is thereby generated on the fixing element (30) about the transverse opening axis (130), which leads to the temporary clamping of the bone anchor head region (91) in the fork head (10).

12. Osteosynthesis device (1) according to one of the preceding claims, **characterized in that** a connecting rod (50) is insertable and fixable on the fork head (10) by means of a locking element (60), thereby achieving an angularly stable clamping between the bone anchor head region (91) and the fork head (10), and the angularly stable clamping with the aid of the locking element (60) and the angularly stable clamping with the aid of the fixing element (30) can be activated independently of one another and is also combinable with one another.

13. Osteosynthesis device (1) according to one of the preceding claims, **characterized in that** the positioning element (40) does not protrude beyond the proximal end of the fork head (10) in the proximal direction (101).

14. Osteosynthesis device (1) according to one of the preceding claims, **characterized in that** the fixing element (30) can assume a rotational position S1 about the transverse opening axis (130), in which a compression force is transmitted on the bone anchor (90), so that the bone anchor (90) is held angularly stable in the ball seat (19), and the fixing element (30) can assume a second rotational position S2, in which the bone anchor (90) is held movably in the ball seat (19).

15. Osteosynthesis device (1) according to one of the preceding claims, **characterized in that** an axial opening (14) for a positioning element (40) has an inner thread at least in sections.

16. Osteosynthesis device (1) according to one of the preceding claims, **characterized in that** the fork head (10) has a thrust piece (20), and the thrust piece has a through opening (28), a distally directed contact region towards the bone anchor head region (91), a proximally directed stave bearing (25), and, in the region of the bone anchor head region (91), a lateral opening or partial cutout (23), in which the fixing element (30) is arranged in a freely movable manner.

## Revendications

1. Dispositif d'ostéosynthèse (1) pour le traitement de la colonne vertébrale, constitué d'une chape (10) en forme de U en vue latérale, qui possède une ouverture de passage (18) et la chape (10) présente dans la direction proximale (101) deux branches de fourche (11, 12) avec un filetage intérieur (16) et dans laquelle une tige de liaison (50) peut être reçue, et dans la chape (10) en direction distale (102) dans l'ouverture de passage (18) est prévue une zone de réception de la tête sphérique (19) et dans laquelle est logé de manière pivotante un ancrage osseux (90), **caractérisé en ce qu'**il est prévu sur la chape (10) une ouverture transversale (13) qui communique avec l'ouverture de passage de la chape (18) par l'intermédiaire d'une découpe de paroi (185) et un élément de fixation (30) est logé dans cette ouverture transversale (13) de manière à pouvoir tourner autour d'un axe de l'ouverture transversale (130) et l'élément de fixation (30) bloque la zone de tête (91) de l'ancrage osseux (90) de manière angulairement stable dans la chape (10) en introduisant un couple de torsion autour de l'axe de l'ouverture transversale (130), dans lequel le centre du filetage interne (16) et le centre de la zone de réception de la tête sphérique (190) définissent un axe central (103), et cet axe central (103) ne croise pas l'axe de l'ouverture transversale (130).

2. Dispositif d'ostéosynthèse (1) selon la revendication 1, **caractérisé en ce qu'**avec la décharge du couple de torsion sur l'élément de fixation (30), la zone de tête (91) de l'ancrage osseux (90) devient mobile dans la chape (10).

3. Dispositif d'ostéosynthèse (1) selon l'une quelconque des revendications précédentes, **caractérisé en ce que** l'axe de l'ouverture transversale (130) est disposé à peu près orthogonalement et à distance de l'axe central (103).

4. Dispositif d'ostéosynthèse (1) selon l'une quelconque des revendications précédentes, **caractérisé en ce que** l'élément de fixation (30) s'étend (185) au moins partiellement dans l'ouverture de passage (18) de la chape (10) et repose contre la zone de la tête sphérique (91) de l'ancrage osseux (90).

5. Dispositif d'ostéosynthèse (1) selon l'une quelconque des revendications précédentes, **caractérisé en ce qu'**une zone de contact (31) de l'élément de fixation (30), orientée radialement vers l'intérieur, s'approche, en vue latérale, d'au moins une partie de la surface extérieure de la zone de tête de l'ancrage osseux (91).

6. Dispositif d'ostéosynthèse (1) selon l'une quelconque des revendications précédentes, **caractérisé en ce qu'**une zone de contact (31) de l'élément de fixation (30) orientée radialement vers l'intérieur présente une rugosité, des encoches ou des dents accrue(s).

7. Dispositif d'ostéosynthèse (1) selon l'une quelconque des revendications précédentes, **caractérisé en ce qu'**une zone de contact (31) est située au-dessus de l'équateur (94) de la zone de tête de l'ancrage osseux (91) pour initier le serrage temporaire dans la direction proximale (101).

8. Dispositif d'ostéosynthèse (1) selon l'une quelconque des revendications précédentes, **caractérisé en ce que** l'élément de fixation (30) est sensiblement en forme de broche.

9. Dispositif d'ostéosynthèse (1) selon l'une quelconque des revendications précédentes, **caractérisé en ce que** la zone de tête (91) de l'ancrage osseux (90) est définie par un rayon R1 et l'élément de fixation (30) à son point le plus épais par un rayon R2, et la distance la plus courte entre l'axe central (103) et l'axe transversal d'ouverture (130) est inférieure à la somme des rayons R1 et R2.

10. Dispositif d'ostéosynthèse (1) selon l'une quelconque des revendications précédentes, **caractérisé en ce que** l'élément de fixation (30) s'étend (32, 33) au-delà d'au moins une paroi latérale de la chape (10) et qu'un levier (39) y est fixé (391, 393) et qu'un actionnement du levier provoque une rotation de l'élément de fixation (30).

11. Dispositif d'ostéosynthèse (1) selon l'une quelconque des revendications précédentes, **caractérisé en ce que** la chape (10) dispose d'une ouverture axiale (14) et un élément de réglage (40) est guidé dans l'ouverture axiale (14) et une force de compression est générée par le réglage de l'élément de réglage (40), qui est transmise directement à l'élément de fixation (30) ou indirectement par l'intermédiaire d'un levier (39), et un couple de torsion est ainsi généré sur l'élément de fixation (30) autour de l'axe de l'ouverture transversale (130), ce qui conduit au serrage temporaire de la zone de tête de l'ancrage osseux (91) dans la chape (10).

12. Dispositif d'ostéosynthèse (1) selon l'une quelconque des revendications précédentes, **caractérisé en ce qu'**une tige de liaison (50) peut être insérée et fixée à la chape (10) avec un élément de verrouillage (60), ce qui permet d'obtenir un serrage angulairement stable entre la zone de tête de l'ancrage osseux (91) et la chape (10), et le serrage angulairement stable à l'aide de l'élément de verrouillage (60) et le serrage angulairement stable à l'aide de l'élément de fixation (30) peuvent être activés indépendamment les uns des autres et peuvent également être combinés les uns avec les autres.

13. Dispositif d'ostéosynthèse (1) selon l'une quelconque des revendications précédentes, **caractérisé en ce qu'**un élément de réglage (40) ne dépasse pas de l'extrémité proximale de la chape (10) dans la direction proximale (101).

14. Dispositif d'ostéosynthèse (1) selon l'une quelconque des revendications précédentes, **caractérisé en ce que** l'élément de fixation (30) peut prendre une position de rotation S1 \autour de l'axe de l'ouverture transversale (130), dans laquelle une force de compression est transmise à l'ancrage osseux (90), de sorte que l'ancrage osseux (90) est maintenu de manière stable angulairement dans le siège sphérique (19) et l'élément de fixation (30) peut prendre une deuxième position de rotation S2, dans laquelle l'ancrage osseux (90) est maintenu mobile dans le siège sphérique (19).

15. Dispositif d'ostéosynthèse (1) selon l'une quelconque des revendications précédentes, **caractérisé en ce qu'**une ouverture axiale (14) pour un élément de réglage (40) possède au moins partiellement un filetage intérieur.

16. Dispositif d'ostéosynthèse (1) selon l'une quelconque des revendications précédentes, **caractérisé en ce que** la chape (10) dispose d'une pièce de pression (20) et la pièce de pression présente une ouverture de passage (28), une zone de contact orientée vers le côté distal par rapport à la zone de tête de l'ancrage osseux (91), un palier de tige (25) orienté vers le côté proximal et, dans la zone de la zone de tête de l'ancrage osseux (29) (91), une ouverture latérale ou une découpe partielle (23) dans laquelle l'élément de fixation (30) est disposé de manière à pouvoir se déplacer librement.
